# EUROPEAN PATENT SPECIFICATION

(11) **EP 1 053 233 B1**
(45) Date of publication and mention of the grant of the patent: **20.08.2003**
(21) Application number: 99904965.3
(22) Date of filing: 05.02.1999
(51) Int. Cl.: C07D 309/08, C07D 401/12, C07C 311/44, A61K 31/35

(54) **HYDROXAMIC AND CARBOXYLIC ACID DERIVATIVES**
HYDROXAMSÄURE- UND CARBONSÄUREDERIVATIVE
DERIVES D'ACIDE HYDROXAMIQUE ET CARBOXYLIQUE

(30) Priority: 06.02.1998 GB 9802620; 08.09.1998 GB 9819570
(43) Date of publication of application: 22.11.2000
(73) Proprietor: Darwin Discovery Limited, Slough, Berkshire SL1 3WE (GB)
(72) Inventor: MONTANA, John, Gary, Celltech Chiroscience Ltd., Abington, Cambridge CB1 6GS (GB); BAXTER, Andrew, Douglas, Celltech Chiroscience Ltd, Abington, Cambridge CB1 6GS (GB); OWEN, David, Alan, Celltech Chiroscience Ltd., Abington, Cambridge CB1 6GS (GB); WATSON, Robert, John, Celltech Chiroscience Ltd., Abington, Cambridge CB1 6GS (GB)
(74) Representative: Perry, Robert Edward
(86) International application number: GB9900374
(87) International publication number: WO99040080

(56) References cited:
- EP-A- 0 780 386
- WO-A-96/35714
- K. JANKOWSKI, ET AL.: "Sur les oxathianne-1,4-one-2" BULLETIN DE L'ACAD MIE POLONAISE DES SCIENCES, S RIE DES SCIENCES CHIMIQUES, vol. 19, no. 11-12, 1971, pages 661-672, XP002081718 Warsaw, PL

## Description

### Field of the Invention

This invention relates to hydroxamic and carboxylic acid derivatives, and to their use in medicine.

### Background to the Invention

Metalloproteinases, including matrix metalloproteinase (MMP), (human fibroblast) collagenase, gelatinase and TNF convertase (TACE), and their modes of action, and also inhibitors thereof and their clinical effects, are described in WO-A-9611209, WO-A-9712902 and WO-A-9719075 MMP inhibitors may also be useful in the inhibition of other mammalian metalloproteinases such as the adamalysin family (or ADAMs) whose members include TNF convertase (TACE) and ADAM-10, which can cause the release of TNFα from cells, and others, which have been demonstrated to be expressed by human articular cartilage cells and also involved in the destruction of myelin basic protein, a phenomenon associated with multiple sclerosis.

Compounds which have the property of inhibiting the action of metalloproteinases involved in connective tissue breakdown, such as collagenase, stromelysin and gelatinase, have been shown to inhibit the release of TNF both *in vitro* and *in vivo*. See Gearing *et al* (1994), Nature 370:555-557; McGeehan *et al* (1994), Nature 370:558-561; GB-A-2268934; and WO-A-9320047. All of these reported inhibitors contain a hydroxamic acid zinc-binding group, as do the imidazole-substituted compounds disclosed in WO-A-9523790. Other compounds that inhibit MMP and/or TNF are described in WO-A-9513289, WO-A-9611209, WO-A-9635687, WO-A-9635711, WO-A-9635712 and WO-A-9635714.

WO-A-9834915 (which may be prior art under Article 54(3) EPC) discloses compounds of formula I (below) wherein B is heterocycloalkyl (optionally substituted by R⁶ or R⁷) bonded through carbon to X.

WO-A-9839315 (which may be prior art under Article 54(3) EPC) discloses compounds of formula I (below) wherein R⁴ is H and R⁵ is unsubstituted C₁₋₆ alkyl, and B is C_{1- 8} alkyl optionally substituted by OR⁷.

### Summary of the Invention

The invention relates to compounds of formula (I), which are useful inhibitors of matrix metalloproteinases and/or TNFα-mediated diseases, including degenerative diseases and certain cancers.

Compounds according to the invention are of the general type represented by formula (I):

R⁶-B-X-(CH₂)ₙ-CR²R³-CR⁴R⁵-COY (I)

wherein
n = 0-1;
X is S(O)₂;
Y is OR¹ or NHOH;
R² and R⁴ are independently H or a group (optionally substituted with R¹⁰) selected from C₁₋₆ alkyl, C₂₋₆ alkenyl, aryl, C₁₋₆ alkyl-aryl, heteroaryl, C₁₋₆ alkyl-heteroaryl, heterocycloalkyl, C₁₋₆ alkyl-heterocycloalkyl, cycloalkyl and C₁₋₆ alkyl-cycloalkyl; and
R¹, R³ and R⁵ are independently H or C₁₋₆ alkyl;
provided that not more than two of R², R³, R⁴ and R⁵ are H; or
any of CR²R³, CR⁴R⁵ and CR²-CR⁴ is a cycloalkyl or heterocycloalkyl ring optionally substituted with R¹⁰ or a group (optionally substituted with R¹⁰) selected from C₁₋₆ alkyl, aryl, C₁₋₆ alkyl-aryl, heteroaryl and C₁₋₆ alkyl-heteroaryl;
B is a divalent group selected from C₁₋₈ alkyl, C₂₋₆ alkenyl and C₂₋₆ alkynyl;
R⁶ is N(R⁷)₂, OR⁷, COR⁷, C(=NOR⁹)R⁷, NR⁷R⁸, S(O)₀₋₂R⁹ or SO₂N(R⁷)₂;
R⁷ is H or a group selected from C₁₋₆ alkyl, aryl, C₁₋₆ alkyl-aryl, heteroaryl, C₁₋₆ alkyl-heteroaryl, cycloalkyl, C₁₋₆ alkyl-cycloalkyl, heterocycloalkyl and C₁₋₆ alkyl-heterocycloalkyl, wherein said group is optionally substituted with R⁹, COR⁹, SO₀₋₂R⁹, CO₂R⁹, OR⁹, CONR¹R⁹, NR¹R⁹, halogen, CN, SO₂NR¹R⁹ or NO₂, and for each case of N(R⁷)₂ the R⁷ groups are the same or different or N(R⁷)₂ is heterocycloalkyl optionally substituted with R⁹, COR⁹, SO₀₋₂R⁹, CO₂R⁹, OR⁹, CONR¹R⁹, NR¹R⁹, halogen, CN, SO₂NR¹R⁹ or NO₂;
R⁸ is COR⁷, CON(R⁷)₂, CO₂R⁹ or SO₂R⁹;
R⁹ is C₁₋₆ alkyl, aryl, C₁₋₆ alkyl-aryl, heteroaryl or C₁₋₆ alkyl-heteroaryl; and
R¹⁰ is OR⁷, COR⁷, CO₂R¹, CON(R⁷)₂, NR⁷R⁸, S(O)₀₋₂R⁹, SO₂N(R⁷)₂, CN, halogen or cycloimidyl (optionally substituted with R¹);
and the salts, solvates, hydrates, N-oxides, protected amino, protected carboxy and protected hydroxamic acid derivatives thereof.

Combinations of substituents and/or variables are only permissible if such combinations result in stable compounds.

### Description of the Invention

Preferred compounds of the invention are those wherein any one or more of the following apply:
Y is NHOH;
R² or R⁴ are optionally substituted C₁₋₆ alkyl, C₁₋₆ alkyl-heteroaryl, or C₁₋₆ alkyl-heterocycloalkyl; or R² and R³, or R⁴ and R⁵, or R² and R⁴ form the said optionally substituted cycloalkyl or heterocycloalkyl groups;
B is C₁₋₈ alkyl substituted with R⁶; and
R⁶ is OR⁷.

The compounds of the Examples are particularly preferred.

It will be appreciated that the compounds according to the invention can contain one or more asymmetrically substituted carbon atoms. The presence of one or more of these asymmetric centres in a compound of formula (I) can give rise to stereoisomers, and in each case the invention is to be understood to extend to all such stereoisomers, including enantiomers and diastereomers, and mixtures including racemic mixtures thereof.

It will further be appreciated that the compounds according to the invention may contain an oxime. This oxime can give rise to geometrical isomers, and in each case the invention is to be understood to extend to all such isomers and mixtures thereof.

As used in this specification, alone or in combination, the term "C₁₋₆alkyl" refers to straight or branched chain alkyl moiety having from one to six carbon atoms, including for example, methyl, ethyl, propyl, isopropyl, butyl, *tert*-butyl, pentyl, hexyl and the like.

The term "C₁₋₈alkyl" refers to straight or branched chain alkyl moiety having from one to eight carbon atoms, including for example, methyl, ethyl, propyl, isopropyl, butyl, *tert*-butyl, pentyl, hexyl, octyl and the like.

The term "C₂₋₆ alkenyl" refers to a straight or branched chain alkyl moiety having two to six carbon atoms and having in addition one double bond, of either E or Z stereochemistry where applicable. This term would include for example, vinyl, 1-propenyl, 1- and 2- butenyl, 2- methyl-2-propenyl etc.

The term "C₂₋₆ alkynyl" refers to a straight or branched chain alkyl moiety having two to six carbon atoms and having in addition one triple bond. This term would include for example, ethynyl, 1-propynyl, 1- and 2- butynyl, 1- methyl-2-butynyl etc.

The term "cycloalkyl" refers to a saturated alicyclic moiety having from three to six carbon atoms and includes for example cyclopropyl, cyclobutyl, cyclopentyl, cyclohexyl and the like.

The term "heterocycloalkyl" refers to a saturated heterocyclic moiety having from two to six carbon atoms and one or more heteroatom from the group N, O, S (or oxidised versions thereof) which may be optionally benzofused at any available position. This includes for example azetidinyl, pyrrolidinyl, tetrahydrofuranyl, piperidinyl, benzodioxole and the like.

The term "aryl" refers to an aromatic carbocyclic radical having a single ring or two condensed rings. This term includes, for example phenyl or naphthyl.

The term "heteroaryl" refers to aromatic ring systems of five to ten atoms of which at least one atom is selected from O, N and S, and includes for example furanyl, thiophenyl, pyridyl, indolyl, quinolyl and the like.

The term "halogen" means fluorine, chlorine, bromine or iodine.

The term "benzofused" refers to the addition of a benzene ring sharing a common bond with the defined ring system.

The term "cycloimidyl" refers to a saturated ring of five to ten atoms containing the atom sequence -C(=O)NC(=O)-. The ring may be optionally benzofused at any available position. Examples include succinimidoyl, phthalimidoyl and hydantoinyl.

The term "optionally substituted" means optionally susbsituted with one or more of the groups specified, at any available position or positions.

The terms "protected amino", "protected carboxy" and "protected hydroxamic acid" mean amino, carboxy and hydroxamic acid groups which can be protected in a manner familiar to those skilled in the art. For example, an amino group can be protected by a benzyloxycarbonyl, *tert*-butoxycarbonyl, acetyl or like group, or may be in the form of a phthalimido or like group. A carboxyl group can be protected in the form of a readily-cleavable ester such as the methyl, ethyl, benzyl or *tert*-butyl ester. A hydroxamic acid may be protected as either N or O-substitued derivatives, such as O-benzyl or O-*tert*-butyldimethylsilyl.

Salts of compounds of formula (I) include pharmaceutically-acceptable salts, for example acid addition salts derived from inorganic or organic acids, such as hydrochlorides, hydrobromides, p-toluenesulphonates, phosphates, sulphates, perchlorates, acetates, trifluoroacetates, propionates, citrates, malonates, succinates, lactates, oxalates, tartrates and benzoates.

Salts may also be formed with bases. Such salts include salts derived from inorganic or organic bases, for example alkali metal salts such as magnesium or calcium salts, and organic amine salts such as morpholine, piperidine, dimethylamine or diethylamine salts.

When the "protected carboxy" group in compounds of the invention is an esterified carboxyl group, it may be a metabolically-labile ester of formula CO₂R¹¹ where R¹¹ may be an ethyl, benzyl, phenethyl, phenylpropyl, α- or β-naphthyl, 2,4-dimethylphenyl, 4-*tert*-butylphenyl, 2,2,2-trifluoroethyl, 1-(benzyloxy)benzyl, 1-(benzyloxy)ethyl, 2-methyl-1-propionyloxypropyl, 2,4,6-trimethylbenzyloxymethyl or pivaloylmethyl group.

Compounds of the general formula (I) may be prepared by any suitable method known in the art and/or by the following processes.

It will be appreciated that, where a particular stereoisomer of formula (I) is required, the synthetic processes described herein may be used with the appropriate homochiral starting material and/or isomers maybe resolved from mixtures using conventional separation techniques (e.g. HPLC).

The compounds according to the invention may be prepared by the following process. In the description and formulae below the groups R¹, R², R³, R⁴, R⁵, R⁶, R⁷, R⁸, R⁹, R¹⁰, R¹¹, B, X and Y are as defined above, except where otherwise indicated. It will be appreciated that functional groups, such as amino, hydroxyl or carboxyl groups, present in the various compounds described below, and which it is desired to retain, may need to be in protected form before any reaction is initiated. In such instances, removal of the protecting group may be the final step in a particular reaction. Suitable protecting groups for such functionality will be apparent to those skilled in the art. For specific details see Greene *et al*, "Protective Groups in Organic Synthesis", Wiley Interscience.

A process for preparing compounds of general formula (I) comprises reacting a compound of formula B-SH (II) with (a) an alkylating agent of formula Z-(CH₂)ₙ-CR²R³-CR⁴R⁵-COY (III) (wherein Z represents a suitable leaving group e.g. a halogen such as bromine, or an alkylsulphonate ester such as methanesulphonate), or (b) (when n = 0 and R⁵ = H) an acrylate of formula CR²R³=CR⁴-COY (IV) or (c) a lactone of the formula (V)

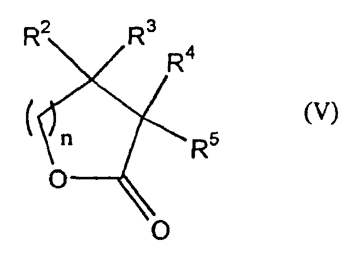

An alternative process for preparing compounds of formula (I) involves reacting a compound of formula B-Z (VI) with a compound of formula Q-S-(CH₂)ₙCR²R³CR⁴R⁵COY (VII), where Z is defined above and Q is a suitable labile group, such as acetyl, in the presence of a strong base such as hexamethyldisilazide in an inert solvent such as tetrahydrofuran. Compounds of formula (VII) may be prepared by reaction of a compound of formula QSH (VIII) with a compound (III), (IV) or (V) as defined above, optionally in the presence of an organic or inorganic base.

Alkylating agents offormula (III) can be obtained in chiral or racemic form. Many ofthese derivatives can be readily obtained from commercially available starting materials using methods known to those skilled in the art (e.g. see WO-A-9005719).

Acrylates of formula (IV) may be prepared by the condensation of a carbonyl compound of the form R⁴CH₂COY (IX) with ketones or aldehydes of formula R²COR³ (X). This reaction may be performed under a variety of conditions known to those skilled in the art, for example under the action of a strong base such as lithium diisopropylamide in an inert solvent such as tetrahydrofuran, to give an intermediate alcohol of the form R²C(OH)R³CHR⁴COY (XI). These alcohols (XI) may or may not be isolated: dehydration may occur *in situ* or be performed separately under appropriate conditions such as aqueous acid to give the desired acrylate (IV).

Lactones of formula (V) may be prepared by chemistry known to those skilled in the art. For example see EP-A-0780386.

Many compounds of formula (II) are available commercially, or may be prepared from compounds of the form B-Z (VI) by standard methods (for example, see WO-A-9611209).

Many compounds offormula (VIII), (VI), (IX) and (X) are available commercially, or may be prepared from compounds available commercially by standard methods. Other substituents described by R², R⁴, or R⁶ or can be introduced by standard chemical transformations known to those skilled in the art.

Compounds of formula (I) may also be prepared by interconversion of other compounds of formula (I). Thus, for example, a compound of formula (I) wherein R² is a C₁₋₆ alkyl group may be prepared by hydrogenation (using palladium on carbon in suitable solvent, such as an alcohol, e.g. ethanol) of a compound of formula (I) wherein R² is a C₂₋₆ alkenyl group. Further, a compound of formula (I) wherein X is S(O)₁₋₂ may be prepared by oxidation of a compound of formula (I) wherein X is S.

Carboxylic acids of general formula (I) (Y=OH) may be converted to other compounds of formula (l) such as esters (Y=OR¹) or hydroxamic acids (Y=NHOH) using methods known to those skilled in the art.

Any mixtures of final products or intermediates obtained can be separated on the basis of the physico-chemical differences of the constituents, in known manner, into the pure final products or intermediates, for example by chromatography, distillation, fractional crystallization, or by formation of a salt if appropriate or possible under the circumstances.

The compounds according to the invention exhibit *in vitro* inhibiting activities with respect to the stromelysins, collagenases and gelatinases. Compounds according to the invention may also exhibit *in vitro* inhibition of membrane shedding events known to be mediated by metalloproteinases, for example, α-APP, ACE, TGF-α, TNF-α, Fas ligand, TNFR-I, TNFR-II, CD30, Il-6R, CD43, CD44, CD16-I, CD16-II, Folate receptor, CD23, or IL-1RII.

The activity and selectivity of the compounds may be determined by use of the appropriate enzyme inhibition test, for example as described in Examples A-M of WO-A-9805635, by the assay for the inhibition of CD23 shedding described in PCT/GB98/03395, or by the following assay of TNF RI shedding.

The potency of the compounds of general formula (I) to act as inhibitors of the production of TNF RI is determined using the following procedure. A 100µM solution of the inhibitor being tested or dilutions thereof is incubated at 37° C in an atmosphere of 5% CO₂ with peripheral blood mononuclear cells (PBMC). PBMC are isolated from buffy coats by standard procedures using Ficoll. A 100µM solution of the inhibitor being tested or dilutions thereof is incubated for 22 hours at 37° C in an atmosphere of 5% CO₂ with 1 x 10⁶/ml PBMC stimulated with LPS. The cells are centrifuged down and the supernatant is assayed for TNF RI using a commercially available ELISA kit (R & D Systems). The activity in the presence of 0.1 mM inhibitor or dilutions thereof is compared to activity in a control devoid of inhibitor and results reported as that inhibitor concentration effecting 50% inhibition of the production of TNF RI.

The compounds of the invention may be used in a method of treatment for patients (including man and/or mammalian animals raised in the dairy, meat or fur industries or as pets) suffering from disorders or diseases which can be attributed to stromelysin as previously described, and more specifically, a method of treatment involving the administration of the matrix metalloproteinase inhibitors of formula (I) as the active constituents.

Accordingly, the compounds of formula (I) can be used among other things in the treatment of osteoarthritis and rheumatoid arthritis, and in diseases and indications resulting from the over-expression of these matrix metalloproteinases such as found in certain metastatic tumour cell lines.

As mentioned above, compounds of formula (I) are useful in human or veterinary medicine since they are active as inhibitors of TNF and MMPs. Accordingly in another aspect, this invention concerns:
The use of compounds (I) in a method of management (by which is meant treatment of prophylaxis) of disease or conditions mediated by TNF and/or MMPs in mammals, in particular in humans, which method comprises administering to the mammal an effective, amount of a compound of formula (I) above, or a pharmaceutically acceptable salt thereof; and
a compound of formula (I) for use in human or veterinary medicine, particularly in the management (by which is meant treatment or prophylaxis) of diseases or conditions mediated by TNF and/or MMPs; and
the use of a compound of formula (I) in the preparation of an agent for the management (by which is meant treatment or prophylaxis) of diseases or conditions mediated by TNF and/or MMPs.

The disease or conditions referred to above include inflammatory diseases, autoimmune diseases, cancer, cardiovascular diseases, diseases involving tissue breakdown such as rheumatoid arthritis, osteoarthritis, osteoporosis, neurodegeneration, Alzheimer's disease, stroke, vasculitis, Crohn's disease, ulcerative colitis, multiple sclerosis, periodontitis, gingivitis and those involving tissue breakdown such as bone resorption, haemorrhage, coagulation, acute phase response, cachexia and anorexia, acute infections, HIV infections, fever, shock states, graft versus host reactions, dermatological conditions, surgical wound healing, psoriasis, atopic dermatitis, epidermolysis bullosa, tumour growth, angiogenesis and invasion by secondary metastases, ophthalmological disease, retinopathy, corneal ulceration, reperfusion injury, migraine, meningitis, asthma, rhinitis, allergic conjunctivitis, eczema, anaphylaxis, restenosis, congestive heart failure, endometriosis, atherosclerosis, endosclerosis and aspirin-independent anti-thrombosis.

Compounds of formula (I) may also be useful in the treatment of pelvic inflammatory disease (PID), age-related macular degeneration and cancer-induced bone resorption. Further, they can be used in the treatment of lung diseases, e.g. selected from cystic fibrosis, adult respiratory distress syndrome (ARDS), emphysema, bronchitis obliterans-organising pneumonia (BOOP), idiopathic pulmonary fibrosis (PIF), diffuse alveolar damage, pulmonary Langerhan's cell granulamatosis, pulmonary lymphangioleiomyomatosis (LAM) and chronic obstructive pulmonary disease (COPD).

For the treatment of rheumatoid arthritis, osteoarthritis, and in diseases and indications resulting from the over-expression of matrix metalloendoproteinases such as found in certain metastatic tumour cell lines or other diseases mediated by the matrix metalloendoproteinases or increased TNF production, the compounds of formula (I) may be administered orally, topically, parenterally, by inhalation spray or rectally in dosage unit formulations containing non-toxic pharmaceutically acceptable carriers, adjuvants and vehicles. The term parenteral as used herein includes subcutaneous injections, intravenous, intramuscular, intrasternal injection or infusion techniques. In addition to the treatment of warm-blooded animals such as mice, rats, horses, cattle, sheep, dogs, cats etc, the compounds of the invention are effective in the treatment of humans.

The pharmaceutical composition containing the active ingredient may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavouring agents, colouring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example corn starch, or alginic acid; binding agents, for example starch, gelatin or acacia, and lubricating agents, for example magnesium stearate, stearic acid or talc. The tablets may be uncoated or they may be coated by known techniques to delay disintegration and absorption in the gastointestinal tract and thereby provide a sustained action over a longer period. For example, a time delay material such as glyceryl monostearate or glyceryl distearate may be employed. They may also be coated by the techniques described in the US Patents 4,256,108; 4,166,452; and 4,265,874, to form osmotic therapeutic tablets for control release.

Formulations for oral use may also be presented as hard gelatin capsules where in the active ingredient is mixed with an inert solid diluent, for example calcium carbonate, calcium phosphate or kaolin, or as soft gelatin capsules wherein the active ingredient is mixed with water or an oil medium, for example peanut oil, liquid paraffin or olive oil.

Aqueous suspensions contain the active materials in admixture with excipients suitable for the manufacture of aqueous suspensions. Such excipients are suspending agents, for example sodium carboxymethylcellulose, methylcellulose, hydroxypropylmethylcellulose, sodium alginate polyvinyl-pyrrolidone, gum tragacanth and gum acacia; dispersing or wetting agents may be a naturally occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such a polyoxyethylene with partial esters derived from fatty acids and hexitol anhydrides, for example polyoxyethylene sorbitan monooleate. The aqueous suspensions may also contain one or more preservatives, for example ethyl, or n-propyl, p-hydroxybenzoate, one or more colouring agents, one or more flavouring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavouring agents may be added to provide a palatable oral preparation. These compositions may be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified, for example sweetening, flavouring and colouring agents, may also be present.

The pharmaceutical compositions of the invention may also be in the form of oil-in-water emulsions. The oily phase may be a vegetable oil, for example olive oil or arachis oil, or a mineral oil, for example liquid paraffin or mixtures of these. Suitable emulsifying agents may be naturally- occurring gums, for example gum acacia or gum tragacanth, naturally-occurring phosphatides, for example soya bean, lecithin, and esters or partial esters derived from fatty acids and hexitol anhydrides, for example sorbitan monooleate and condensation products of the said partial esters with ethylene oxide, for example polyoxyethylene sorbitan monooleate. The emulsions may also contain sweetening and flavouring agents.

Syrups and elixirs may be formulated with sweetening agents, for example glycerol, propylene glycol, sorbitol or sucrose. Such formulations may also contain a demulcent, a preservative and flavouring and colouring agents. The pharmaceutical compositions may be in the form of a sterile injectable aqueous or oleagenous suspension. This suspension may be formulated according to the known art using those suitable dispersing or wetting agents and suspending agents which have been mentioned above. The sterile injectable preparation may also be in a sterile injectable solution or suspension in a non-toxic parenterally-acceptable diluent or solvent, for example as a solution in 1,3-butane diol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

The compounds of formula (I) may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

For topical use, creams, ointments, jellies, solutions or suspensions, etc containing the compounds of Formula (I) are employed. For the purposes of this specification, topical application includes mouth washes and gargles.

Dosage levels of the order of from about 0.05 mg to about 140 mg per kilogram of body weight per day are useful in the treatment of the above- indicated conditions (about 2.5 mg to about 7 g per patient per day). For example, inflammation may be effectively treated by the administration of from about 0.01 to 50 mg ofthe compound per kilogram of body weight per day (about 0.5 mg to about 3.5 g per patient per day).

The amount of active ingredient that may be combined with the carrier materials to produce a single dosage form will vary depending upon the host treated and the particular mode of administration. For example, a formulation intended for the oral administration of humans may vary from about 5 to about 95% of the total composition. Dosage unit forms will generally contain between from about 1 mg to about 500 mg of active ingredient.

It will be understood, however, that the specific dose level for any particular patient will depend upon a variety of factors including the activity of the specific compound employed, the age, body weight, general health, sex, diet time of administration, route of administration, rate of excretion, drug combination and the severity of the particular disease undergoing therapy.

The following Examples illustrate the invention. The following abbreviation is used:
RT = Room Temperature DMF = Dimethylformamide THF = Tetrahydrofuran

### Intermediate 1 2,7-Dioxaspiro[3.5]nonan-1-one

Was prepared according to the procedure outlined in EP-A-0780386, as a colourless oil (5.2 g).

### Intermediate 2 1-Bromo-3-(4-chlorophenoxy)propane

Sodium hydride (3.3 g, 60% dispersion in oil) was added to a solution of 4-chlorophenol (9.0 g) in DMF (30 ml) at 0°C and the mixture was stirred for 30 min, then 1,3-dibromopropare (22.5 g, 1.5 eq) was added. The mixture was stirred at room temperature for 18 h, then added to water (300 ml) and extracted with ether. The solvent was washed with 5% NaOH and brine, dried over MgSO₄ and evaporated and the residue purified on silica gel, eluting with 1:1 ether/hexane, to give the title compound (13.5 g) as a colourless oil.
TLC R_{f} 0.73 (ether).

### Intermediate 3 1-Acetylsulfanyl-3-(4-chlorophenoxy)propane

A solution of Intermediate 2 (6.0 g) in DMF (50 ml) was treated with potassium thioacetate (5.7 g) at room temperature for 3 h, then the brown solution was added to 5% sodium bicarbonate solution and extracted with ether (3 x 200 ml). The solvent was washed with water (200 ml) and brine, dried over MgSO₄ and evaporated to give the title compound (5.50 g) as a beige solid.
TLC R_{f} 0.54 (ether).

### Intermediate 4 Methyl 4-Iodomethyl-tetrahydropyran-4-carboxylate

To a stirred solution of methyl tetrahydropyran-4-carboxylate (3.00 g) in anhydrous THF (50 ml) under nitrogen at 0°C was added a solution of lithium diisopropylamide (2.0 M, 10.9 ml). After 30 minutes, diiodomethane (8.36 g) was added and the mixture was allowed to warm up to RT and then stirred for 1 h. The mixture was poured onto water (50 ml) and extracted with ether (3 x 40 ml). The combined ether layers were washed with water (2 x 20 ml), aqueous hydrochloric acid (2 M, 20 ml), water (20 ml) and brine (20 ml), dried (MgSO₄) and evaporated under reduced pressure. The residue was purified on silica gel, eluting with 3:1 hexane/ether, to give the title compound (3.11 g) as a colourless oil.
TLC R_{f} 0.26 (3:1 hexane/ether)

### Intermediate 5 Methyl 4-Acetylsulfanylmethyl-tetrahydropyran-4-carboxylate

A solution of Intermediate 4 (3.05 g) in DMF (20 ml) was treated with potassium thioacetate (1.47 g) at room temperature for 18 h, then the brown solution was added to 5% sodium bicarbonate solution and extacted with ether (3 x 200 ml). The solvent was washed with water (200 ml) and brine, dried over MgSO₄ and evaporated to give the title compound (2.42 g) as a white solid.
TLC R_{f} 0.44 (1:1 hexane/ether).

### Example 1 4-((3-(4-Chlorophenoxy)propylsulfanyl)methyl)-tetrahydropyran-4-carboxylic Acid

A solution of Intermediate 3 (0.80 g) in methanol was treated with a solution of sodium hexamethyldisilazide (1 M in THF, 3.3 ml) at 0°C and the resulting mixture was stirred for 2 h, then a solution of Intermediate 1 (460 mg) in methanol was added. The mixture was stirred for 18 h, then evaporated and the residue dissolved in water and washed with diethyl ether. The aqueous phase was acidified with citric acid and extracted with dichloromethane; the organic layer was then washed with brine, dried over MgSO₄ and evaporated to give the title compound (0.12 g) as a colourless oil.
TLC R_{f} 0.54 (ether)

### Example 2 Methyl 4-(3-Hydroxypropylsulfanylmethyl)tetrahydropyran-4-carboxylate

To a stirred solution of Intermediate 5 (2.50 g) in methanol (100 ml) under nitrogen at 0°C was added a solution of sodium hexamethyldisilazide (1M in THF, 11.3 ml) and the resulting mixture was stirred for 10 mins, then a solution of 3-bromopropanol (1.5 g) was added. The mixture was stirred for 6 h, then evaporated and the residue treated with water (100 ml) and extracted with dichloromethane (4 x 50 ml). The combined dichloromethane extracts were washed with water (2 x 50 ml), brine (50 ml), dried over MgSO₄ and evaporated. The residue was purified on silica gel eluting with 2:1 ethyl acetate/hexane, to give the title compound (2.41 g) as a colourless oil.
TLC R_{f} 0.26 (2:1 ethyl acetate/hexane).

### Example 3 Methyl 4-((3-(4-Methoxyphenoxy)propylsulfanyl)methyl)-tetrahydropyran-4-carboxylate

To a stirred solution of Example 2 (500 mg) and triphenylphosphine (528 mg) in dry THF (25 ml) at 0°C under nitrogen, was added diethylazodicarboxylate (351 mg). After 5 min a solution of 4-methoxyphenol (250 mg) in THF was added. The mixture was stirred for 2 h with the temperature rising to RT, then poured into ether (100 ml) and washed with water (2 x 25 ml), sodium hydroxide solution (1M; 25 ml), brine (25ml) and dried over MgSO₄. Evaporation and purification on silica gel eluting with 2:1 hexane/ethyl acetate, gave the title compound (415 mg) as a colourless oil.
TLC R_{f} 0.25 (2:1 hexane/ethyl acetate).

Similarly prepared were:

### Example 4 Methyl 4-((3-(4-Phenylphenoxy)propylsulfanyl)methyl)-tetrahydropyran-4-carboxylate

From Example 2 (500 mg) and 4-phenylphenol (343 mg) as a white solid (298 mg). TLC R_{f} 0.45 (2:1 hexane/ethyl acetate).

### Example 5 Methyl 4-((3-(3-Pyridyloxy)propylsulfanyl)methyl)-tetrahydropyran-4-carboxylate

From Example 2 (500 mg) and 3-hydroxypyridine (191 mg) as a colourless oil (360 mg).
TLC R_{f} 0.19 (2:1 ethyl acetate/hexane).

### Example 6 Methyl 4-((3-(4-Methoxyphenoxy)propylsulfonyl)methyl)-tetrahydropyran-4-carboxylate

To a stirred solution of Example 3 (400 mg) in methanol (10 ml) at RT was added a solution of Oxone (1.04 g) in water (20 ml). Stirring was continued for 18 h before diluting with water (50 ml) and extracting with dichloromethane (4 x 25 ml). The combined organic layers were washed with water (25 ml), brine (25 ml), dried over MgSO₄ and evaporated to give the title compound (385 mg) as a colourless oil.
TLC R_{f} 0.33 (2:1 ethyl acetate/hexane).

Similarly prepared were:

### Example 7 Methyl 4-((3-(4-Phenylphenoxy)propylsulfonyl)methyl)-tetrahydropyran-4-carboxylate

From Example 4 (290 mg) as a white solid (303 mg).
TLC R_{f} 0.33 (2:1 ethyl acetate/hexane).

### Example 8 Methyl 4-((3-(3-Pyridyloxy)propylsulfonyl)methyl)-tetrahydropyran-4-carboxylate

From Example 5 (325 mg) as a colourless oil (350 mg).
TLC R_{f} 0.15 (ethyl acetate).

### Example 9 4-((3-(4-Chlorophenoxy)propylsulfonyl)methyl)-tetrahydropyran-4-carboxylic Acid

Oxone (0.42 g) was added to a solution of Example 1 (0.12 g) in methanol (10 ml) and water (5 ml) at room temperature and the mixture was stirred for 3 h. The mixture was then evaporated to half volume, diluted with water (5 ml) and extracted with dichloromethane (2 x 20 ml). The organic layer was then washed with brine, dried over MgSO₄ and evaporated and the residue purified by flash column chromatography on silica gel, eluting with ethyl acetate/acetic acid (99:1) to give the title compound (70 mg) as a white solid.
TLC R_{f} 0.70
MS 376 (M⁺)

### Example 10 4-((3-(4-Methoxyphenoxy)propylsulfonyl)methyl)-tetrahydropyran-4-carboxylic Acid

To a stirred solution of Example 6 (380 mg) in methanol (30 ml) was added a solution of lithium hydroxide (206 mg) in water (10 ml). The mixture was refluxed for 1.5 h, cooled to RT and diluted with water (50 ml). The aqueous mixture was washed with ether (2 x 20 ml), acidified (2M HCI, pH1) and extracted with ethyl acetate (5 x 25ml). The combined ethyl acetate fractions were washed with water (2 x 20 ml), brine (20ml), dried over MgSO₄ and evaporated to give the title compound (266 mg) as a white solid.
TLC R_{f} 0.10 (ethyl acetate)
MS 372 (M⁺)

Similarly prepared were:

### Example 11 4-((3-(4-Phenylphenoxy)propylsulfonyl)methyl)-tetrahydropyran-4-carboxylic Acid

From Example 7 (300 mg) as a white solid (203 mg).
TLC R_{f} 0.15 (ethyl acetate)
MS 418 (M⁺)

### Example 12 4-((3-(3-Pyridyloxy)propylsulfonyl)methyl)tetrahydropyran-4-carboxylate

From Example 8 (325 mg) as a white solid (103 mg).
TLC R_{f} 0.10(ethyl acetate)
MS 343 (M⁺)

### Example 13 4-((3-(4-Chlorophenoxy)propylsulfonyl)methyl)-tetrahydropyran-4-carboxylic Acid N-Hydroxy Amide

EDC (40 mg) was added to a solution of Example 9 (75 mg) in dichloromethane (20 ml), followed by *tert*-butyldimethylsilylhydroxylamine (32 mg) and *N,N*-dimethylaminopyridine (2 mg), and the resulting clear solution was stirred at room temperature for 3 h. The mixture was then washed with water, saturated bicarbonate and brine, dried over MgSO₄ and evaporated. The residue was dissolved in a minimum amount of fresh dichloromethane and 1M aqueous hydrochloric acid in ether (2 ml) was added. The solution was stirred for 10 min, then evaporated and the residue purified by chromatography on silica, eluting with ethyl acetate, to give the title compound (30 mg) as a colourless glass.
TLC R_{f} 0.27 (EtOAc)
MS 391 (M⁺)

### Example 14 4-((3-(4-Methoxyphenoxy)propylsulfonyl)methyl)-tetrahydropyran-4-carboxylic Acid N-Hydroxy Amide

To a stirred solution of Example 10 (250 mg) in dichloromethane (15 ml) containing a drop of DMF was added oxalyl chloride (256 mg). The mixture was stirred for 1 h before removing the solvent under reduced pressure. The residue was treated three times with a mixture of dichloromethane/hexane (1:1; 50 ml) and evaporated to dryness. The residue was dissolved in THF (15 ml) and treated with an aquoeus solution of hydroxylamine (50 wt%; 0.21 ml). The mixture was stirred for 1 h before diluting with water (10 ml) and extracting with ethyl acetate (3 x 10 ml). The combined ethyl acetate extracts were washed with saturated sodium bicarbonate solution (20 ml), water (20 ml), brine (20 ml), dried over MgSO₄ and evaporated to yield the title compound (145 mg) as a colourless solid.
TLC R_{f} 0.60 (9:1 dichloromethane/methanol)
MS 387 (M⁺)

Similarly prepared was:

### Example 15 4-((3-(4-Phenylphenoxy)propylsulfonyl)methyl)-tetrahydropyran-4-carboxylic Acid N-Hydroxy Amide

From Example 11 (180 mg) as a white solid (124 mg).
TLC R_{f} 0.42 (9:1 dichloromethane/methanol)
MS 433 (M⁺)

## Claims

1. A compound of formula (I)
R⁶-B-S(O)₂-(CH₂)ₙ-CR²R³-CR⁴R⁵-COY (I)
wherein
n is 0 or 1;
Y is OR¹ or NHOH;
R² and R⁴ are independently H or a group (optionally substituted with R¹⁰) selected from C₁₋₆ alkyl, C₂₋₆ alkenyl, aryl, C₁₋₆ alkyl-aryl, heteroaryl, C₁₋₆ alkyl-heteroaryl, heterocycloalkyl, C₁₋₆ alkyl-heterocycloalkyl, cycloalkyl and C₁₋₆ alkyl-cycloalkyl;
R¹, R³ and R⁵ are independently H or C₁₋₆ alkyl;
provided that not more than two of R², R³, R⁴ and R⁵ are H; or
any of CR²R³, CR⁴R⁵ and CR²-CR⁴ is a cycloalkyl or heterocycloalkyl ring optionally substituted with R¹⁰ or a group (optionally substituted with R¹⁰) selected from C₁₋₆ alkyl, aryl, C₁₋₆ alkyl-aryl, heteroaryl and C₁₋₆ alkyl-heteroaryl;
B is a divalent group selected from C₁₋₈ alkyl, C₂₋₆ alkenyl and C₂₋₆ alkynyl;
R⁶ is N(R⁷)₂, OR⁷, COR⁷, C(=NOR⁹)R⁷, NR⁷R⁸, S(O)₀₋₂R⁹ or SO₂N(R⁷)₂;
R⁷ is H or a group selected from C₁₋₆ alkyl, aryl, C₁₋₆ alkyl-aryl, heteroaryl, C₁₋₆ alkyl-heteroaryl, cycloalkyl, C₁₋₆ alkyl-cycloalkyl, heterocycloalkyl and C₁₋₆ alkyl-heterocycloalkyl, wherein said group is optionally substituted with R⁹, COR⁹, SO₀₋₂R⁹, CO₂R⁹, OR⁹, CONR¹R⁹, NR¹R⁹, halogen, CN, SO₂NR¹R⁹ or NO₂, and for each case of N(R⁷)₂ the R⁷ groups are the same or different, or N(R⁷)₂ is heterocycloalkyl optionally substituted with R⁹, COR⁹, SO₀₋₂R⁹, CO₂R⁹, OR⁹, CONR¹R⁹, NR¹R⁹, halogen, CN, SO₂NR¹R⁹ or NO₂;
R⁸ is COR⁷, CON(R⁷)₂, CO₂R⁹ or SO₂R⁹;
R⁹ is C₁₋₆ alkyl, aryl, C₁₋₆ alkyl-aryl, heteroaryl or C₁₋₆ alkyl-heteroaryl;
R¹⁰ is OR⁷, COR⁷, CO₂R¹, CON(R⁷)₂, NR⁷R⁸, S(O)₀₋₂R⁹, SO₂N(R⁷)₂, CN, halogen or cycloimidyl (optionally substituted with R¹);
aryl is an aromatic carboxylic radical having a single ring or two condensed rings;
heteroaryl is an aromatic ring system of 5 to 10 atoms of which at least one is N, O or S;
heterocycloalkyl is a saturated heterocyclic moiety having 2 to 6 C atoms and one or more N, O or S atoms, any N atom optionally being oxidised, and which is optionally benzofused; and
cycloalkyl is a saturated alicyclic moiety having 3 to 6 C atoms; or
a salt, solvate, hydrate, N-oxide or protected amino, protected carboxy or protected hydroxamic acid derivative thereof.

2. A compound of claim 1, wherein R² or R⁴ is optionally substituted C₁₋₆ alkyl, C₁₋₆ alkyl-heteroaryl, or C₁₋₆ alkyl-heterocycloalkyl; or CR²R³, CR⁴R⁵ or CR²-CR⁴ forms the said optionally substituted ring.

3. A compound of claim 1 or claim 2, wherein B is C₁₋₈ alkyl.

4. A compound of any preceding claim, wherein R⁶ is OR⁷.

5. A compound of claim 1, which is
4-((3-(4-chlorophenoxy)propylsulfonyl)methyl)tetrahydropyran-4-carboxylic acid or its N-hydroxy amide.

6. A compound of claim 1, which is
methyl 4-((3-(4-methoxyphenoxy)propylsulfonyl)methyl)tetrahydropyran-4-carboxylate,
methyl 4-((3-(4-phenylphenoxy)propylsulfonyl)methyl)tetrahydropyran-4-carboxylate,
methyl 4-((3-(3-pyridyloxy)propylsulfonyl)methyl)tetrahydropyran-4-carboxylate,
4-((3-(3-pyridyloxy)propylsulfonyl)methyl)tetrahydropyran-4-carboxylic acid,
4-((3-(4-methoxyphenoxy)propylsulfonyl)methyl)tetrahydropyran-4-carboxylic acid or its N-hydroxy amide, or
4-((3-(4-phenylphenoxy)propylsulfonyl)methyl)tetrahydropyran-4-carboxylic acid N-hydroxy amide.

7. A compound of any of claims 1 to 6, which is in the form of a single enantiomer or diastereomer.

8. A pharmaceutical composition for use in therapy, comprising a compound as defined in any preceding claim, and a pharmaceutically-acceptable diluent or carrier.

9. Use of a compound of any of claims 1 to 7, for the manufacture of a medicament for the treatment of a condition selected from cancer, inflammation and inflammatory diseases, tissue degeneration, periodontal disease, ophthalmological disease, dermatological disorders, fever, cardiovascular effects, haemorrhage, coagulation and acute phase response, cachexia, anorexia, acute infection, HIV infection, shock states, graft versus host reactions, autoimmune disease, reperfusion injury, meningitis, migraine and aspirin-independent anti-thrombosis.

10. Use of a compound of any of claims 1 to 7, for the manufacture of a medicament for the treatment of a condition selected from tumour growth, angiogenesis, tumour invasion and spread, metastases, malignant ascites and malignant pleural effusion.

11. Use of a compound of any of claims 1 to 7, for the manufacture of a medicament for the treatment of a condition selected from cerebral ischaemia, ischaemic heart disease, rheumatoid arthritis, osteoarthritis, osteoporosis, asthma, multiple sclerosis, neurodegeneration, Alzheimer's, atherosclerosis, stroke, vasculitis, Crohn's disease and ulcerative colitis.

12. Use of a compound of any of claims 1 to 7, for the manufacture of a medicament for the treatment of a condition selected from corneal ulceration, retinopathy and surgical wound healing.

13. Use of a compound of any of claims 1 to 7, for the manufacture of a medicament for the treatment of a condition selected from psoriasis, atopic dermatitis, chronic ulcers and epidermolysis bullosa.

14. Use of a compound of any of claims 1 to 7, for the manufacture of a medicament for the treatment of a condition selected from periodontitis and gingivitis.

15. Use of a compound of any of claims 1 to 7, for the manufacture of a medicament for the treatment of a condition selected from rhinitis, allergic conjunctivitis, eczema and anaphylaxis.

16. Use of a compound of any of claims 1 to 7, for the manufacture of a medicament for the treatment of a condition selected from restenosis, congestive heart failure, endometriosis, atherosclerosis and endosclerosis.

17. Use of a compound of any of claims 1 to 7, for the manufacture of a medicament for the treatment of a condition selected from pelvic inflammatory disease (PID), age-related macular degeneration and cancer-induced bone resorption.

18. Use of a compound of any of claims 1 to 7, for the manufacture of a medicament for the treatment of a condition which is a lung disease.

19. Use according to claim 18, wherein the lung disease is selected from cystic fibrosis adult respiratory distress syndrome (ARDS), emphysema, bronchitis obliterans-organising pneumonia (BOOP), idiopathic pulmonary fibrosis (PIF), diffuse alveolar damage, pulmonary Langerhan's cell granulamatosis, pulmonary lymphangioleiomyomatosis (LAM) and chronic obstructive pulmonary disease (COPD).

## Patentansprüche

1. Verbindung der Formel (I)
R⁶-B-S(O)₂-(CH₂)ₙ-CR²R³-CR⁴R⁵-COY (I)
worin
n 0 oder 1 ist;
Y OR¹ oder NHOH ist;
R² und R⁴ unabhängig H oder eine Gruppe (gegebenenfalls substituiert mit R¹⁰) ausgewählt aus C₁₋₆-Alkyl, C₂₋₆-Alkenyl, Aryl, C₁₋₆-Alkylaryl, Heteroaryl, C₁₋₆-Alkylheteroaryl, Heterocycloalkyl, C₁₋₆-Alkylheterocycloalkyl, Cycloalkyl und C₁₋₆-Alkylcycloalkyl sind;
R¹, R³ und R⁵ unabhängig H oder C₁₋₆-Alkyl sind;
mit der Maßgabe, dass nicht mehr als zwei von R², R³, R⁴ und R⁵ H sind; oder
einer von CR²R³, CR⁴R⁵ und CR²-CR⁴ ein Cycloalkyl- oder Heterocycloalkylring, gegebenenfalls substituiert mit R¹⁰ oder einer Gruppe (gegebenenfalls substituiert mit R¹⁰) ausgewählt aus C₁₋₆-Alkyl, Aryl, C₁₋₆-Alkylaryl, Heteroaryl und C₁₋₆-Alkylheteroaryl ist;
B eine zweiwertige Gruppe ausgewählt aus C₁₋₈-Alkyl, C₂₋₆-Alkenyl und C₂₋₆-Alkinyl ist;
R⁶ N(R⁷)₂, OR⁷, COR⁷, C(=NOR⁹)R⁷, NR⁷R⁸, S(O)₀₋₂R⁹ oder SO₂N(R⁷)₂ ist;
R⁷ H oder eine Gruppe ausgewählt aus C₁₋₆-Alkyl, Aryl, C₁₋₆-Alkylaryl, Heteroaryl, C₁₋₆-Alkylheteroaryl, Cycloalkyl, C₁₋₆-Alkylcycloalkyl, Heterocycloalkyl und C₁₋₆-Alkylheterocycloalkyl ist, wobei die Gruppe gegebenenfalls substituiert ist mit R⁹, COR⁹, SO₀₋₂R⁹, CO₂R⁹, OR⁹, CONR¹R⁹, NR¹R⁹, Halogen, CN, SO₂NR¹R⁹ oder NO₂ und für jeden Fall von N(R⁷)₂ die Gruppen R⁷ gleich oder verschieden sind oder N(R⁷)₂ ein Heterocycloalkyl, gegebenenfalls substituiert mit R⁹, COR⁹, SO₀₋₂R⁹, CO₂R⁹, OR⁹, CONR¹R⁹, NR¹R⁹, Halogen, CN, SO₂NR¹R⁹ oder NO₂ ist;
R⁸ COR⁷, CON(R⁷)₂, CO₂R⁹ oder SO₂R⁹ ist;
R⁹ C₁₋₆-Alkyl, Aryl, C₁₋₆-Alkylaryl, Heteroaryl oder C₁₋₆-Alkylheteroaryl ist;
R¹⁰ OR⁷, COR⁷, CO₂R¹, CON(R⁷)₂, NR⁷R⁸, S(O)₀₋₂R⁹, SO₂N(R⁷)₂, CN, Halogen oder Cycloimidyl (gegebenenfalls substituiert mit R¹) ist,
Aryl ein aromatischer Carboxylrest mit einem einzelnen Ring oder zwei kondensierten Ringen ist,
Heteroaryl ein aromatisches Ringsystem mit 5 bis 10 Atomen ist, von denen mindestens eines N, O oder S ist;
Heterocycloalkyl eine gesättigte heterocyclische Gruppe mit 2 bis 6 C-Atomen und einem oder mehreren N-, O- oder S-Atomen ist, wobei ein N-Atom gegebenenfalls oxidiert ist, welche gegebenenfalls benzokondensiert ist; und
Cycloalkyl eine gesättigte alicyclische Gruppe mit 3 bis 6 C-Atomen ist; oder
ein Salz, ein Solvat, ein Hydrat, ein N-Oxid- oder geschütztes Amino-, geschütztes Carboxy- oder geschütztes Hydroxamsäure-Derivat davon.

2. Verbindung nach Anspruch 1, worin R² oder R⁴ gegebenenfalls substituiertes C₁₋₆-Alkyl, C₁₋₆-Alkylheteroaryl oder C₁₋₆-Alkylheterocycloalkyl sind oder CR²R³, CR⁴R⁵ oder CR²-CR⁴ den genannten gegebenenfalls substituierten Ring bilden.

3. Verbindung nach Anspruch 1 oder Anspruch 2, worin B C₁₋₈-Alkyl ist.

4. Verbindung nach irgendeinem vorhergehenden Anspruch, worin R⁶ OR⁷ ist.

5. Verbindung nach Anspruch 1, welche
4-((3-(4-Chlorphenoxy)propylsulfonyl)methyl)tetrahydropyran-4-carbonsäure oder deren N-Hydroxyamid ist.

6. Verbindung nach Anspruch 1, welche
Methyl-4-((3-(4-methoxyphenoxy)propylsulfonyl)methyl)tetrahydropyran-4-carboxylat,
Methyl-4-((3-(4-phenylphenoxy)propylsulfonyl)methyl)tetrahydropyran-4-carboxylat,
Methyl-4-((3-(3-pyridyloxy)propylsulfonyl)methyl)tetrahydropyran-4-carboxylat,
4-((3-(3-Pyridyloxy)propylsulfonyl)methyl)tetrahydropyran-4-carbonsäure,
4-((3-(4-Methoxyphenoxy)propylsulfonyl)methyl)tetrahydropyran-4-carbonsäure oder deren N-Hydroxyamid oder
4-((3-(4-Phenylphenoxy)propylsulfonyl)methyl)tetrahydropyran-4-carbonsäure-N-hydroxyamid ist.

7. Verbindung nach irgendeinem der Ansprüche 1 bis 6, welche in Form eines Enantiomers oder Diastereomers vorliegt.

8. Pharmazeutische Zusammensetzung zur Verwendung in der Therapie umfassend eine Verbindung wie in irgendeinem vorhergehenden Anspruch definiert und ein pharmazeutisch verträgliches Verdünnungsmittel oder einen pharmazeutisch verträglichen Träger.

9. Verwendung einer Verbindung nach irgendeinem der Ansprüche 1 bis 7 für die Herstellung eines Arzneimittels zur Behandlung eines Zustands, der ausgewählt ist aus Krebs, Entzündung und Entzündungskrankheiten, Gewebedegeneration, Wurzelhautentzündung, Augenkrankheiten, dermatologischen Störungen, Fieber, kardiovaskulären Effekten, Hämorrhagie, Gerinnung und Akutphasen-Reaktion, Kachexie, Anorexie, akuter Infektion, HIV-Infektion, Schockzuständen, Transplantat-Wirt-Reaktionen, Autoimmunkrankheiten, Reperfusionsschädigung, Meningitis, Migräne und Aspirin-unabhängiger Thromboseprophylaxe.

10. Verwendung einer Verbindung nach irgendeinem der Ansprüche 1 bis 7 für die Herstellung eines Arzneimittels zur Behandlung eines Zustands, der ausgewählt ist aus Tumorwachstum, Angiogenese, Tumorinvasion und -ausbreitung, Metastasen, malignem Ascites und malignem Pleuraerguss.

11. Verwendung einer Verbindung nach irgendeinem der Ansprüche 1 bis 7 für die Herstellung eines Arzneimittels zur Behandlung eines Zustands, der ausgewählt ist aus zerebraler Ischämie, ischämischer Herzerkrankung, rheumatoider Arthritis, Osteoarthritis, Osteoporose, Asthma, Multipler Sklerose, Neurodegeneration, Alzheimer-Krankheit, Arteriosklerose, Schlaganfall, Vaskulitis, Morbus Crohn und ulzeröser Kolitis.

12. Verwendung einer Verbindung nach irgendeinem der Ansprüche 1 bis 7 für die Herstellung eines Arzneimittels zur Behandlung eines Zustands, der ausgewählt ist aus Homhautgeschwüren, Retinopathie und Heilung einer Operationswunde.

13. Verwendung einer Verbindung nach irgendeinem der Ansprüche 1 bis 7 für die Herstellung eines Arzneimittels zur Behandlung eines Zustands, der ausgewählt ist aus Psoriasis, atopischer Dermatitis, chronischen Geschwüren und Epidermolysis bullosa.

14. Verwendung einer Verbindung nach irgendeinem der Ansprüche 1 bis 7 für die Herstellung eines Arzneimittels zur Behandlung eines Zustands, der ausgewählt ist aus Periodontitis und Gingivitis.

15. Verwendung einer Verbindung nach irgendeinem der Ansprüche 1 bis 7 für die Herstellung eines Arzneimittels zur Behandlung eines Zustands, der ausgewählt ist aus Rhinitis, allergischer Konjunktivitis, Exzem und Anaphylaxie.

16. Verwendung einer Verbindung nach irgendeinem der Ansprüche 1 bis 7 für die Herstellung eines Arzneimittels zur Behandlung eines Zustands, der ausgewählt ist aus Restenose, Stauungsherzinsuffizienz, Endometriose, Arteriosklerose und Endosklerose.

17. Verwendung einer Verbindung nach irgendeinem der Ansprüche 1 bis 7 für die Herstellung eines Arzneimittels zur Behandlung eines Zustands, der ausgewählt ist aus Salpingitis (PID, "pelvic inflammatory disease"), altersbedingter Makuladegeneration und durch Krebs-induzierter Knochenresorption.

18. Verwendung einer Verbindung nach irgendeinem der Ansprüche 1 bis 7 für die Herstellung eines Arzneimittels zur Behandlung eines Zustands, der eine Lungenkrankheit ist.

19. Verwendung nach Anspruch 18, worin die Lungenkrankheit ausgewählt ist aus zystischer Fibrose, Atemnotsyndrom des Erwachsenen (ARDS, "adult respiratory distress syndrome"), Emphysem, Bronchiolitis obliteransorganisierender Pneumonie (BOOP), idiopathischer Lungenfibrose (PIF, "idiopathic pulmonary fibrosis"), diffusem Alveolarschaden, Langerhanszell-Lungengranulomatose, Lungen-Lymphangioleiomyomatose (LAM) und chronisch-obstruktiver Lungenerkrankung (COLD).

## Revendications

1. Composé de formule (I) :
R⁶-B-S(O)₂-(CH₂)ₙ-CR²R³-CR⁴R⁵-COY (I)
où
n est égal à 0 ou 1 ;
Y est OR¹ ou NHOH ;
R² et R⁴ sont, de façon indépendante, H ou un groupe (substitué de façon optionnelle par R¹⁰) choisi parmi des restes alkyle en C₁₋₆, alcényle en C₂₋₆, aryle, (alkyl en C₁₋₆)aryle, hétéroaryle, (alkyl en C₁₋₆)hétéroaryle, hétérocycloalkyle, (alkyl en C₁₋₆)hétérocycloalkyle, cycloalkyle et (alkyl en C₁₋₆)cycloalkyle ;
R¹, R³ et R⁴ sont, de façon indépendante, H ou un reste alkyle en C₁₋₆ ;
sous réserve que deux au plus des R², R³, R⁴ et R⁵ soient un H ; ou
un quelconque des CR²R³, CR⁴R⁵ et CR²-CR⁴ est un cycle cycloalkyle ou hétérocycloalkyle, substitué de façon optionnelle par R¹⁰ ou un groupe (substitué de façon optionnelle par R¹⁰) choisi parmi des restes alkyle en C₁₋₆, aryle, (alkyl en C₁₋₆)aryle, hétéroaryle et (alkyl en C₁₋₆)hétéroaryle ;
B est un groupe divalent choisi parmi alkyle en C₁₋₈, alcényle en C₂₋₆ et alcynyle en C₂₋₆ ;
R⁶ est N(R⁷)₂, OR⁷, COR⁷, C(=NOR⁹)R⁷, NR⁷R⁸, S(O)₀₋₂R⁹ ou SO₂N(R⁷)₂;
R⁷ est un H ou un groupe choisi parmi alkyle en C₁₋₆, aryle, (alkyl en C₁₋₆)aryle, hétéroaryle, (alkyl en C₁₋₆)hétéroaryle, cycloalkyle, (alkyl en C₁₋₆)cycloalkyle, hétérocycloalkyle et (alkyl en C₁₋₆)hétérocycloalkyle, ledit groupe étant substitué de façon optionnelle par R⁹, COR⁹, S(O)₀₋₂R⁹, CO₂R⁹, OR⁹, CONR¹R⁹, NR¹R⁹, halogène, CN, SO₂NR¹R⁹ ou NO₂ et, pour chaque cas de N(R⁷)₂, les groupes R⁷ sont identiques ou différents, ou N(R⁷)₂ est un reste hétérocycloalkyle substitué de façon optionnelle par R⁹, COR⁹, SO₀₋₂R⁹, CO₂R⁹, OR⁹, CONR¹R⁹, NR¹R⁹, halogène, CN, SO₂NR¹R⁹ ou NO₂ ;
R⁸ est un reste COR⁷, CON(R⁷)₂, CO₂R⁹ ou S(O)₂R⁹ ;
R⁹ est un reste alkyle en C₁₋₆, aryle, (alkyl en C₁₋₆)aryle, hétéroaryle ou (alkyl en C₁₋₆)hétéroaryle ;
R¹⁰ est OR⁷, COR⁷, CO₂R¹, CON(R⁷)₂, NR⁷R⁸, S(O)₀₋₂R⁹, SO₂N(R⁷)₂, CN, halogène ou cycloimidyle (substitué de façon optionnelle par R¹) ;
aryle est un reste carbocyclique aromatique ayant un seul cycle ou deux cycles condensés ;
hétéroaryle est un système cyclique aromatique de 5 à 10 atomes dont au moins un est un N, un O ou un S ;
hétérocycloalkyle est un fragment hétérocyclique saturé ayant 2 à 6 atomes de carbone et un ou plusieurs atomes N, O ou S, tout atome N pouvant être de façon optionnelle oxydé, et qui est de façon optionnelle condensé à un benzo ; et
cycloalkyle est un fragment alicyclique saturé ayant 3 à 6 atomes de carbone ;
ou
un sel, solvate, hydrate, N-oxyde de ce composé ou un dérivé de celui-ci à fonction amino protégée, carboxy protégée ou acide hydroxamique protégée.

2. Composé selon la revendication 1, dans lequel R² ou R⁴ est un reste alkyle en C₁₋₆, (alkyl en C₁₋₆)hétéroaryle ou (alkyl en C₁₋₆)hétérocycloalkyle substitué de façon optionnelle ; ou bien CR²R³, CR⁴R⁵ ou CR²-CR⁴ forme ledit cycle substitué de façon optionnelle.

3. Composé selon la revendication 1 ou la revendication 2, dans lequel B est un groupe alkyle en C₁₋₈.

4. Composé selon l'une quelconque des revendications précédentes, dans lequel R⁶ est OR⁷.

5. Composé selon la revendication 1, qui est
l'acide 4-((3-(4-chlorophénoxy)propylsulfonyl)méthyl)tétrahydropyran-4-carboxylique ou son N-hydroxyamide.

6. Composé selon la revendication 1, qui est
le 4-((3-(4-méthoxyphénoxy)propylsulfonyl)méthyl)tétrahydropyran-4-carboxylate de méthyle,
le 4-((3-(4-phénylphénoxy)propylsulfonyl)méthyl)tétrahydropyran-4-carboxylate de méthyle,
le 4-((3-(3-pyridyloxy)propylsulfonyl)méthyl)tétrahydropyran-4-carboxylate de méthyle,
l'acide 4-((3-(3-pyridyloxy)propylsulfonyl)méthyl)tétrahydropyran-4-carboxylique,
l'acide 4-((3-(4-méthoxyphénoxy)propylsulfonyl)méthyl)tétrahydropyran-4-carboxylique ou son N-hydroxyamide ou
le N-hydroxyamide de l'acide 4-((3-(4-phénylphénoxy)propylsulfonyl)méthyl)-tétrahydropyran-4-carboxylique.

7. Composé selon l'une quelconque des revendications 1 à 6, sous la forme d'un énantiomère unique ou d'un diastéréoisomère.

8. Composition pharmaceutique utile en thérapie, comprenant un composé défini dans l'une quelconque des revendications précédentes et un diluant ou un support pharmaceutiquement acceptable.

9. Utilisation d'un composé selon l'une quelconque des revendications 1 à 7, pour la fabrication d'un médicament pour le traitement d'une pathologie choisie parmi le cancer, l'inflammation et les maladies inflammatoires, une dégénérescence tissulaire, une parodontopathie, une maladie ophtalmologique, des troubles dermatologiques, une fièvre, des effets cardiovasculaires, une hémorragie, une coagulation et une réponse de phase aiguë, une cachexie, une anorexie, une infection aiguë, une infection par le VIH, des états de choc, des réactions greffon contre hôte, une maladie autoimmune, un trouble de reperfusion, une méningite, une migraine et un état anti-thrombotique indépendant de l'aspirine.

10. Utilisation d'un composé selon l'une quelconque des revendications 1 à 7, pour la fabrication d'un médicament pour le traitement d'une pathologie choisie parmi une croissance de tumeur, une angiogénèse, un envahissement et une extension de tumeur, des métastases, des ascites malins et un épanchement pleural malin.

11. Utilisation d'un composé selon l'une quelconque des revendications 1 à 7, pour la fabrication d'un médicament pour le traitement d'une pathologie choisie parmi une ischémie cérébrale, une cardiopathie ischémique, la polyarthrite rhumatoïde, l'arthrose, l'ostéoporose, l'asthme, la sclérose en plaque, une neurodégénérescence, la maladie d'Alzheimer, l'athérosclérose, un accident vasculaire cérébral, une angéite, une maladie de Crohn et une rectocolite hémorragique.

12. Utilisation d'un composé selon l'une quelconque des revendications 1 à 7, pour la fabrication d'un médicament pour le traitement d'une pathologie choisie parmi une ulcération de la cornée, une rétinopathie et une cicatrisation de plaie chirurgicale.

13. Utilisation d'un composé selon l'une quelconque des revendications 1 à 7, pour la fabrication d'un médicament pour le traitement d'une pathologie choisie parmi le psoriasis, une dermite atopique, des ulcères chroniques ou une épidermolyse bulleuse.

14. Utilisation d'un composé selon l'une quelconque des revendications 1 à 7, pour la fabrication d'un médicament pour le traitement d'une pathologie choisie parmi une parodontite et une gingivite.

15. Utilisation d'un composé selon l'une quelconque des revendications 1 à 7, pour la fabrication d'un médicament pour le traitement d'une pathologie choisie parmi une rhinite, une conjonctivite allergique, un eczéma et une anaphylaxie.

16. Utilisation d'un composé selon l'une quelconque des revendications 1 à 7, pour la fabrication d'un médicament pour le traitement d'une pathologie choisie parmi une resténose, une insuffisance cardiaque, une endométriose, une athérosclérose et une endosclérose.

17. Utilisation d'un composé selon l'une quelconque des revendications 1 à 7, pour la fabrication d'un médicament pour le traitement d'une pathologie choisie parmi un syndrome inflammatoire pelvien (PID), une dégénérescence maculaire liée à l'âge et une résorption osseuse d'origine cancéreuse.

18. Utilisation d'un composé selon l'une quelconque des revendications 1 à 7, pour la fabrication d'un médicament pour le traitement d'une pathologie qui est une maladie des poumons.

19. Utilisation selon la revendication 18, dans laquelle la maladie des poumons est choisie parmi une mucoviscidose, un syndrome de détresse respiratoire de l'adulte (ARDS), un emphysème, une bronchite oblitérante avec pneumonie organisée (BOOP), une fibrose pulmonaire iodiopathique (PIF), une lésion alévolaire diffuse, une granulomatose pulmonaire des cellules de Langerhans, une lymphangioléiomyomatose (LAM) pulmonaire et une maladie pulmonaire obstructive chronique (COPD).
